(19) Europäisches Patentamt

European Patent Office

Office européen des brevets

(11)  **EP 4 523 218 B1**

(12)  # EUROPEAN PATENT SPECIFICATION

(45) Date of publication and mention
of the grant of the patent:
**21.01.2026 Bulletin 2026/04**

(21) Application number: **23725714.2**

(22) Date of filing: **11.05.2023**

(51) International Patent Classification (IPC):
$G16H\ 15/00^{(2018.01)}$  $G16H\ 30/40^{(2018.01)}$
$G16H\ 50/30^{(2018.01)}$  $A61B\ 5/00^{(2006.01)}$
$A61C\ 7/00^{(2006.01)}$  $G06F\ 3/04815^{(2022.01)}$
$G06F\ 3/0482^{(2013.01)}$  $A61C\ 9/00^{(2006.01)}$
$G16H\ 50/50^{(2018.01)}$  $G16H\ 50/70^{(2018.01)}$

(52) Cooperative Patent Classification (CPC):
**A61B 5/4547; A61C 9/0053; G06F 3/04815;
G06F 3/0482; G16H 30/40; G16H 50/50;
G16H 50/70;** A61B 5/0013; A61B 5/0062;
A61B 5/0088; A61B 5/743; A61B 2576/00

(86) International application number:
**PCT/EP2023/062555**

(87) International publication number:
**WO 2023/217939 (16.11.2023 Gazette 2023/46)**

(54) **METHOD AND SYSTEM FOR IDENTIFYING ISLANDS OF INTEREST**

VERFAHREN UND SYSTEM ZUM IDENTIFIZIEREN VON INTERESSENSINSELN

PROCÉDÉ ET SYSTÈME D'IDENTIFICATION D'ÎLES D'INTÉRÊT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **12.05.2022 EP 22173065**

(43) Date of publication of application:
**19.03.2025 Bulletin 2025/12**

(73) Proprietor: **3Shape A/S
1060 Copenhagen K (DK)**

(72) Inventors:
• **ALALOUF, Daniella**
**1060 Copenhagen K (DK)**
• **STOUSTRUP, Asger**
**1060 Copenhagen K (DK)**
• **CEBOV, Aleksandar**
**1060 Copenhagen K (DK)**
• **MIHOV, Filip**
**1060 Copenhagen K (DK)**

(74) Representative: **Guardian
IP Consulting I/S
Diplomvej, Building 381
2800 Kgs. Lyngby (DK)**

(56) References cited:
WO-A1-2020/182880        WO-A1-2021/113501
US-A1- 2008 118 131      US-A1- 2012 268 463
US-A1- 2013 271 462      US-A1- 2017 308 626
US-A1- 2021 128 282      US-A1- 2022 148 263

• **THOMAS FORGIONE ET AL: "Impact of 3D
bookmarks on navigation and streaming in a
networked virtual environment", PROCEEDINGS
OF THE 7TH INTERNATIONAL CONFERENCE ON
MULTIMEDIA SYSTEMS, MMSYS '16, 10 May
2016 (2016-05-10), New York, New York, USA,
pages 1 - 10, XP055473484, ISBN:
978-1-4503-4297-1, DOI: 10.1145/
2910017.2910607**

## Description

### TECHNICAL FIELD

The disclosure relates to a computer-implemented method and system for identifying islands of interest on a virtual 3D dental model and, more particularly, to obtaining a plurality of islands on the 3D dental model and displaying the 3D dental model.

### BACKGROUND

It is known that various 3D scanning techniques exist, and that virtual 3D dental models reflecting intraoral situation of a subject (patient) can be generated by use of known 3D scanning techniques. For example, by use of an intraoral 3D scanner it is possible to obtain 3D scan data, and therefrom to generate a virtual 3D dental model which accurately represents objects in a mouth of the subject, for example teeth and gingiva. By processing the 3D scan data indications of dental conditions can be determined and presented on the generated 3D dental model. The 3D dental model can be displayed, for example on a display, together with the indications of dental conditions. A user, for example a dentist, can manually interact with the generated 3D model and visually inspect the determined indications of dental conditions. However, this method of inspection relies on the manual effort by the user who needs to carefully search for indications of dental conditions on the 3D dental model. For example, indications of dental conditions may be scattered across multiple sections of the 3D dental model or even be in sections which are not directly in the field of view of the user. It is therefore cumbersome to manually identify and inspect all indications of dental conditions on the 3D dental model.

Document D1 (WO 2020/182880 A1) discloses a method for generating a digital three-dimensional model representing development in dental condition for a tooth is disclosed. The method includes obtaining, at different timepoints, a first digital 3D model of a patient's set of teeth including first texture data and a second digital 3D model of the patient's set of teeth including second texture data. The first digital 3D model including the first texture data and second digital 3D model including the second texture data are placed in a common texture space by uniformizing texture. Lastly, the digital three-dimensional model representing development in dental condition is generated based on a comparison of the first texture data and the second texture data of corresponding regions in the first digital 3D model and the second digital 3D model placed in the common texture space.

Document D2 (THOMAS FORGIONE ET AL: "Impact of 3D bookmarks on navigation and streaming in a networked virtual environment", PROCEEDINGS OF THE 7TH INTERNATIONAL CONFERENCE ON MULTIMEDIA SYSTEMS, MMSYS '16, 10 May 2016 (2016-05-10)) discloses a 3D bookmark in a networked virtual environment (NVE) that provides a navigation aid, allowing the user to move quickly from its current viewpoint to a bookmarked viewpoint by simply clicking on the bookmark. Validation of the positive impact that 3D bookmarks have in easing navigation in a 3D scene is performed.

Document D3 (US 2012/268463 A1) discloses systems, methods, and design software allow for easy and efficient rendering, displaying, navigation, and/or sharing of computer generated designs and models. One or more implementations allow a user to share the geometry and the graphical attributes of a three-dimensional model without sharing the source file or the proprietary details of the source file. In particular, the one or more implementations provide the ability to capture the geometry and the graphical attributes of a three-dimensional model, and share then captured geometry with others.

Document D4 (US 2017/308626 A1) discloses a virtual reality system comprising a virtual reality headset, one or more position tracking sensors, and a computerized architectural design environment. The position tracking sensors can identify a relative location of the user with respect to a physical space. The computerized architectural design environment can comprise a computer-implemented method for displaying a three-dimensional view of an architectural design to a user through the one or more virtual reality components.

Document D5 (US 2013/271462 A1) discloses a new and useful multi-component method of creating computer models of real estate properties for the purpose of conducting virtual and interactive real estate tours. The invention comprises a 3D mapping component where a 3D model of a property is created using a 3D modeling program, and a 3D walkthrough component where panoramic photos are created and interconnected to create a virtual tour of the interior spaces of the property.

Document D6 (US 2008/118131 A1) discloses a method for processing computed tomography (CT) datasets comprises identifying regions of interest (ROIs) within a CT dataset is provided. The ROIs are ranked based on a comparison to at least one predetermined parameter. The ranking determines a level of importance for the ROIs with respect to each other. A list of the ROIs is provided on a display, the list indicating the ROIs based on an associated level of importance. The ROIs are selectable with a user interface.

Document D7 (WO 2021/113501) discloses a method for intraoral imaging generating one or more output imaging signals from an intraoral probe and acquiring multimodal image content from intraoral surface locations according to tissue response from the one or more imaging signals and associating spatial coordinates to the acquired multimodal image content. A surface contour of the patient dentition is generated by stitching the acquired multimodal image content and preserving the association of spatial coordinates with the stitched multimodal image content. Tooth outlines for one or more teeth are generated from the generated surface contour and the generated outlines arranged as a dental chart representing a spatial ordering of the one or more teeth and of supporting gum tissue adjacent to the teeth. The dental chart is populated by analyzing the acquired multimodal image content and associating the analysis to positions on the dental chart according to the preserved association of spatial coordinates and is displayed.

Document D8 (US 2021/128282 A1) discloses intraoral scanning methods and apparatuses for generating a three-dimensional model of a subject's intraoral region (e.g., teeth) including both surface features and internal features. These methods and apparatuses may be used for identifying and evaluating lesions, caries and cracks in the teeth. Any of these methods and apparatuses may use minimum scattering coefficients and/or segmentation to form a volumetric model of the teeth.

Based on the above, there is a clear need to develop a method which allows simple identification and overview of indications of dental conditions on 3D dental models.

## SUMMARY

The invention is set out in the appended set of claims.

According to an embodiment, a computer-implemented method for identifying islands of interest on a 3D dental model is disclosed.

The term "3D dental model" in the present disclosure refers to a virtual 3D dental model.

In the context of the present disclosure term "island" may be understood as a surface area, an area of interest or a volume on the 3D dental model, indicating presence of a dental condition. The island on the 3D dental model may take any shape and may be characterized by its surface size or its volume. The island may be comprised of individual surface or volume units of the 3D dental model, further referred to as regions. The method according to the disclosure comprises the step of receiving at least a first dental 3D scan data of a subject. This data may initially be obtained by scanning oral cavity of the subject with an intraoral scanner at a certain point in time. The first dental 3D scan data may be a series of 2D images used to generate the 3D dental model. The first dental 3D scan data may comprise texture data such as color data or fluorescence data. An example of the intraoral scanner is Trios 4 by 3 Shape. The at least first dental 3D scan data of the subject may be obtained alternatively by 3D-scanning a dental cast, created by taking a dental impression of the subject, with a 3D scanner. The at least first dental 3D scan data may be received by a data processing unit where it can be processed further.

The method further comprises generating a 3D dental model based on the received at least first dental 3D scan data of the subject. The 3D dental model, generated from the at least first dental 3D scan data of the subject, accurately represents intraoral situation of the subject which means that it is an accurate representation of subject's upper and lower set of teeth as well as surrounding gingiva. The 3D dental model may be represented, for example, by a 3D mesh. The 3D mesh may be comprised of plurality of triangles or other mesh-forming units. Individual points on the 3D mesh are generally referred to as vertices. Two vertices may be connected to each other by straight lines called edges. Three edges can form a triangle of the 3D mesh, also known as a facet. The 3D dental model may, in another example, be represented by a point cloud or in any other way of representing 3D objects. Generated 3D dental model may be displayed to a user, for example on a display of a computer or a tablet.

The method according to the disclosure additionally comprises a step of identifying a plurality of regions on the 3D dental model wherein each region of the plurality of regions indicates presence of at least one dental condition. A region may be considered as an individual building element of the 3D mesh, such as a vertex or a facet, indicating presence of a dental condition. Regions on the generated 3D dental model may indicate presence of at least one dental condition which the subject may suffer from. Dental condition in the context of the disclosure may comprise tooth wear, caries presence, gingivitis, or any combination thereof. For example, if the subject suffers from caries presence on a section of a canine tooth in the upper jaw, this dental condition would be reflected in a region or plurality of regions on the generated 3D dental model corresponding to the actual diseased section of the canine tooth. The first dental 3D scan data, out of which the 3D dental model is generated, may comprise the information about the type and the location of the dental condition. The presence of at least one dental condition may be identified by an identification function, as explained later.

Additionally, a severity factor value is determined for each region of the plurality of regions, wherein the severity factor value indicates severity of the dental condition. In case of caries presence, the severity factor value may be obtained by a scoring function to determine a caries score. Here, the severity factor value may take a form of scalar values 0 to 6, according to International Caries Detection and Assessment System (ICDAS) caries severity classification. In the case of tooth wear, the severity factor value may be expressed in millimeters cubed indicating the volume amount of tooth mass loss. In case of gingivitis, the severity factor value may be expressed using Modified Gingival Index (MGI) classification with scalar values 0 to 4.

According to the method of the disclosure, each region of the plurality of regions on the generated 3D dental model is marked with a visual indicator wherein the visual indicator is selected based on determined severity factor value for said region. For example, a region indicating presence of caries, for which low severity factor has been determined, may be visually marked on the 3D dental model with yellow color. The determined severity factor value may be a scalar number 1 or 2 according to ICDAS scoring system, and this may also be referred to as "initial carries". Similarly, a region indicating presence of caries, for which high severity factor value has been determined, may be visually marked on the 3D dental model with red color. Purpose of such marking is to focus user's attention to the parts of the 3D dental model needing inspection. The user may thus first focus on inspecting parts of the 3D dental model with identified regions marked with red color, potentially representing a serious development of the at least one dental condition. The user may interact with the 3D dental model via a user interface by rotation, translation, zooming in or zooming out, for the purpose to obtain a clear and unobstructed view of the part of the 3D dental model of interest. However, in case the regions indicating presence of the at least one dental condition is scattered across the 3D dental model, the user may have to perform multiple manual operations interacting with the 3D dental model, in order to bring the regions of interest into a field of view for diagnostic purposes. With this process there is a high likelihood the user does not notice regions on the periphery of the 3D dental model or regions being blocked from the field of view by other parts of the 3D dental model. The field of view in the context of the disclosure may be understood as a direction that is perpendicular to the surface of the display.

The method further comprises a step of obtaining a plurality of islands by grouping the plurality of regions on the 3D dental model such that each island comprises neighboring regions indicating presence of the at least one dental condition. As mentioned, the term "island" may be understood as a surface area, an area of interest or a volume on the 3D dental model. The island may thus be formed by a plurality of neighboring regions all of which indicate presence of the at least one dental condition. For example, some regions of an island may indicate presence of caries while other regions of the same island may indicate presence of tooth wear. Alternatively, all regions of one island may indicate presence of the same dental condition. One common feature all regions of the same island have in common is that they neighbor at least one other region of that island. For example, where the 3D dental model is represented by a triangular mesh, at least two regions of the island share at least one common edge of a facet in the mesh. By grouping the neighboring regions together into islands, a significant advantage is achieved because globalized information on subject's dental conditions is created. This globalized information is advantageous over localized information represented only by the regions marked with visual indicators as the complexity of the 3D dental model is reduced, allowing the user easier inspection of dental conditions.

The method comprises, additional to receiving the first dental 3D scan data of the subject, receiving a second dental 3D scan data of the same subject, the second dental 3D scan data being obtained later in time compared to the first dental 3D scan data. The second dental 3D scan data may be obtained by scanning oral cavity of the subject with an intraoral scanner or it may be obtained in an alternative manner by 3D-scanning a dental cast created by taking a dental impression of the subject with a 3D scanner. This second dental 3D scan data may be obtained several days or several months after obtaining the first dental 3D scan data. It is common to obtain the second dental 3D scan data one year after obtaining the first dental 3D scan data. The second dental 3D data may be received by the data processing unit together with the first dental 3D scan data, or separately. The 3D dental model in this case can be generated based on the received first and second dental 3D scan data. For example, a first 3D dental model may be generated based on the first dental 3D scan data and a second 3D dental model may be generated based on the second dental 3D scan data. The 3D dental model may then be generated by overlapping the first and the second 3D dental model. It can also be said that the two 3D dental models are mutually aligned. This is particularly advantageous as it may be possible to directly compare the second dental 3D scan data with the first dental 3D scan data of the subject. When displayed, the 3D dental model represents direct comparison of the first and second dental 3D scan data obtained at different times. The 3D dental model may visualize a change in a dental condition that has occurred in the time between obtaining the first and second dental 3D scan data. The 3D dental model may, in this case, be referred to as scan comparison. For example, initial caries determined in a certain region from the first dental 3D scan data may progress to a moderate caries in that region, determined from the second dental 3D scan data. Another example is tooth wear, where a change in tooth wear can be indicated on the displayed 3D dental model. The change in tooth wear, for a specific region on the 3D dental model, may be indicated by different colors depending on the level of the wear.

Further, the method comprises displaying a user interface in form of a preview list, wherein the preview list comprises a plurality of list members. Each list member is associated with an island from the plurality of islands such that the user can navigate between the islands on the 3D dental model by navigating between the list members. This feature is particularly useful as it allows the user to have simple access to all identified islands on the 3D dental model because all the islands are associated to the preview list members. Additionally, by navigating between the list members, via a user interface, the user can also navigate between the islands on the 3D dental model. As can be seen in further detail later, this feature allows the user to easily detect and inspect the at least one dental condition associated with the islands. Thus, the user is credibly assisted in performing a task of interacting with the 3D dental model for the purpose of inspecting dental conditions.

According to an embodiment, the preview list comprising the plurality of list members may be automatically generated. The plurality of list members may be created such that each list member is associated with an island from the plurality of islands. It is therefore an advantage that the list members associated with islands are generated without user interaction where each island is determined through algorithmic analysis of the 3D dental model.

Further, the method comprises, when a list member in the preview list is selected by the user, automatically displaying the island on the 3D dental model associated with the selected list member. The preview list may be displayed with navigation buttons such as a "Previous" and "Next" button, allowing the user to navigate between different list members. The preview list may also be displayed in a way that allows the user to click on different list member without using the navigation buttons. Once the user has pressed the navigation button or clicked on a specific list member, the corresponding island is automatically displayed on the 3D dental model. Alternatively or additionally, the user may navigate between the list members with a mouse "scroll" button or any other suitable user input method.

The automatic displaying of the island associated with the selected list member may comprise automatically adjusting the 3D dental model such that the associated island is brought into the field of view. The field of view may be considered as a direction perpendicular to the display surface. The island may be brought into the field of view by fitting a plane to vertices of the regions comprising the island, obtaining a normal of the plane and bringing the normal to coincide with the field of view. Automatically adjusting the 3D dental model may comprise rotating, translating, magnifying and/or making transparent at least a part of the 3D dental model. Adjustment of the 3D dental model may occur as follows. As a first step, zoom out may be performed. Secondly, rotation and/or translation movements may be performed and finally, zoom in to the specific area of interest may be performed. These steps can be performed automatically.

During the zoom in step, transparency to the surface surrounding the island may be applied. Similarly, during the zoom out step, transparence may be removed so that the whole 3D dental model is being viewed without transparency applied. The steps of adding or removing the transparency can be performed automatically.

In one embodiment, the method further comprises automatically sorting the list members in the preview list based on a sorting criterion, wherein the sorting criterion can be island surface area. Thus, a list member corresponding to the island with the largest surface area may appear as the first list member, a list member corresponding to the island with the second largest surface area may appear as the second list member, and so on. The sorting criterion can be an island significance factor value which can be defined as a function of the island surface area and severity factor values of regions comprising the island. The island significance factor value may be a scalar value. A list member corresponding to the island with the highest island significance factor value may appear as the first list member in the preview list. Additionally or alternatively, the sorting criterion can comprise both the island surface area and the island significance factor value. Thus, the user is credibly assisted in performing a task of inspecting the most relevant area of interest in a most efficient manner. For example, the user may inspect first the island with the largest surface area, or the island with the highest island significance factor value. **In** this way, efficiency in detecting a specific dental condition and designing an appropriate treatment plan is maximized.

The island surface area can be calculated as sum of areas of individual regions comprising the island. **In** case a region is a triangle of the 3D mesh with known coordinates for vertices A, B and C, then area of the region may be calculated according to the commonly used vector product formula for calculating triangle area: Area = ½ $|\overrightarrow{AB} \times \overrightarrow{AC}|$

The list member corresponding to the island with the highest significance factor value can be automatically identified and the 3D dental model may be adjusted so that the island corresponding to the identified list member is brought into the field of view. This means that the user can immediately look at and inspect the most serious dental condition represented in the 3D dental model of the subject's intraoral situation. For example, in case of a severe caries on one of the molar teeth, the 3D dental model would automatically be adjusted so that the island representing caries on the molar tooth is brought directly into the field of view of the user, for example the dentist. Alternatively, the list member corresponding to the island with the largest surface area can be automatically identified and the 3D dental model may be adjusted so that the island corresponding to the identified list member is brought into the field of view.

Method according to an embodiment comprises, when the list member from the preview list is selected by the user, adjusting the 3D dental model so that the island corresponding to the selected list member is brought into the field of view. Additionally, displaying the island surface value for the island corresponding to the selected list member may be comprised. This feature allows the user to select a list member from the preview list, visually inspect the corresponding island on the 3D dental model and get information about the island surface value, for example as displayed square millimeter value next to the island.

Besides the displayed island surface value in square millimeters, a tooth number and/or the order number of the island in the preview list may be displayed additionally. For example, the tooth number may be a number from 1 to 16. The order number of the island may be no. 1 if the island is the first island in the preview list. The user may select a dental treatment based on the information on the island surface value.

Additionally or alternatively, the method according to an embodiment may comprise displaying at least one infrared (IR) image of a tooth or a plurality of teeth associated with the displayed island. The at least one infrared image may be a 2D infrared image.

In an embodiment, marking each region of the plurality of regions with the visual indicator comprises coloring each region of the plurality of regions. For example, regions having high severity factor value can be colored in red color. This is beneficial to the user as it is possible to easily detect red regions on the 3D dental model which require prioritized inspection. Regions having lower severity factor value may be colored in a different shade of red color or, for example, yellow color or green color. Other colors may be used too. An island thus may comprise regions having different severity factor values and may therefore be colored in multiple different colors or in different shades of a same color.

However, if the user wishes to inspect the island in detail, the color may be obstructing the view. Therefore, in one embodiment, displaying the island further comprises removing the visual indicator from each region comprising the island such that the user has unobstructed view of the island on the 3D dental model.

In one embodiment, displaying the island may comprise blurring a part of the 3D dental model other than the displayed island. This may be advantageous as it assists the user with the task of inspecting the 3D dental model more efficiently. Alternatively, instead of blurring the part of the 3D dental model other than the displayed island, the part of the 3D dental model other than the displayed island may be rendered with a lower resolution compared to a part of the 3D dental model corresponding to the displayed island.

Alternatively or additionally, automatically adjusting the 3D dental model may comprise fading out the part of the 3D dental model other than the island brought into the field of view. Fading out may be achieved by changing transparency of the part of the 3D dental model other than the displayed island or coloring this part of the 3D dental model in a dark color for example. This may be advantageous as it assists the user with the task of inspecting the 3D dental model more efficiently.

Alternatively, instead of fading out the part of the 3D dental model other than the island brought into the field of view, the island brought into the field of view may be colored in a darker color or a brighter color compared to the rest of the 3D dental model.

The island brought into the field of view may be configured to blink in order to draw attention from the user and thereby assists the user with the task of inspecting the 3D dental model more efficiently.

In one embodiment automatically adjusting the 3D dental model may comprise only displaying outlines of the island brought into the field of view. The displayed outlines may be colored depending on the determined severity factor value for the island.

According to an embodiment the level of severity of the at least one dental condition may be a high level of severity if the determined severity factor value is higher than a first threshold. If the determined severity factor value lies between the first threshold and a second threshold the level of severity may be a medium level of severity. If the determined severity factor value lies below the second threshold the level of severity may be a low level of severity. In case of tooth wear as dental condition, the severity factor value may directly represent the amount of tooth wear, or loss of tooth substance, expressed in millimeters cubed. Tooth wear can be observed on the 3D dental model which is generated by overlapping or aligning the first 3D dental model and the second 3D dental model. Tooth wear or loss of tooth substance from the first 3D dental model to the second 3D dental model can be visualized with a heat map overlaying the 3D dental model, for example. The heat map may indicate distances between corresponding vertices of the first and the second 3D dental model, when the models are aligned. In one example the first threshold may be 0.8 millimeters and the second threshold may be 0.3 millimeters. The severity factor value in case of tooth wear can be represented in alternative manner as well. For example, known Tooth Wear Index scoring (TWI) can be used, with overall scores from 0 to 4 assigned to the regions.

In one embodiment the method further comprises copying the island significance factor value into a digital dental chart. Additionally or alternatively, the method comprises copying the island surface area into the digital dental chart. The digital dental chart may for example be in format of Universal Numbering System where teeth in the upper jaw are represented with numbers 1 to 16 and teeth in the lower jaw are represented with numbers 17 to 32. The island significance factor and/or the island surface area of the specific tooth in the 3D dental model can be copied to the corresponding tooth representation in the digital dental chart.

In one example, the method further comprises, when the list member from the preview list is selected by the user, the step of automatically adjusting the 3D dental model so that the island corresponding to the selected list member is brought into the field of view.

Additionally, the method comprises the step of calculating an island volumetric difference value for the island corresponding to the selected list member and displaying the island volumetric difference value for the island corresponding to the selected list member. The island volumetric difference can be understood as a change in volume of an island on the 3D dental model, where the 3D dental model is generated based on the first 3D dental model and the second 3D dental model. The island volumetric difference may thus indicate tooth volume loss or gain for a specific tooth, when the first 3D dental model and the second 3D dental model are aligned. The island volumetric difference can be displayed in millimeters cubed. An example of a gain in volume can be presence of dental calculus or tartar, which is a form of a hardened dental plaque, because tooth volume increases over time due to dental calculus. Another example of a volume gain is a dental filling. Tooth wear is an example of volume loss because tooth volume decreases over time due to tooth wear. One way to calculate the island volumetric difference can be to calculate signed volume of tetrahedrons which are formed by joining the vertices of triangular facets in the island with an arbitrary point. Summing up the obtained volumes results in a desired value of the island volumetric difference. The changes in the volume can be highlighted by using the heat map. Each vertex in the island may be colored with a color based on its distance to the opposing surface. The colors on a single facet can then be interpolated between the vertices to achieve the full surface coloring.

In an embodiment, the method may comprise calculating the island volumetric difference for the island corresponding to the selected list member. The island may represent a difference between surfaces of the aligned first 3D dental model and the second 3D dental model and may thus be delimited by two island surfaces, the island on the first 3D dental model and the island on the second 3D dental model. The island volumetric difference measure may quantify the difference between the aligned first 3D dental model and the second 3D dental model. A value of this volumetric difference may be determined and may give a significant insight into severity of the patient's dental condition, for example it may indicate how much tooth volume has been lost due to caries or how much dental calculus has been accumulated in a time span between obtaining the first 3D and the second dental 3D scan data.

Generating the 3D dental model based on the received at least first and second dental 3D data of the subject may comprise:

- generating the first 3D dental model based on the first dental 3D scan data,
- generating the second 3D dental model based on the second dental 3D scan data,
- overlapping the first and the second 3D dental model.

Calculating the island volumetric difference may comprise identifying boundary facets of the island on the second 3D dental model. These boundary facets may be identified as facets not having three neighbors but instead having only one or two neighboring facets indicating presence of the dental condition.

Further, for each identified boundary facet, a closest facet on the first 3D dental model may be identified. The identified closest facets on the first 3D dental model may be mutually connected to form a closed path defining an inner surface and an outer surface on the first 3D dental model. Facets of the inner surface may be defined, for example by running a Flood-Fill algorithm where a facet inside the inner surface is identified and the inner surface area is "flooded" until the closed path is reached.

Further, the identified closest facets on the first 3D dental model may be connected to the corresponding boundary facets on the second 3D dental model to define a closed volume which represents the desired island volumetric difference. This process of connecting the facets may also be referred to as "stitching" process.

In one example, "stitching" may comprise use of a dynamic programming approach. For example, "a" is an array of all boundary facets on the inner surface of the first 3D dental model and "b" is an array of all boundary facets on the island on the second 3D dental model. Further if a[0] and b[0] are the closest pair of points between the two boundaries then the two-dimensional recurrence relation for the dynamic programming approach may be given by:

$f(i, j)$ = max($f(i - 1, j)$ + $dist(a[i - 1], b[j])$), ($f(i, j - 1)$ + $dist(a[i], b[j - 1])$), when $(i, j) \neq (0,0)$ and $dist(a[0], b[0])$ when $(i, j)$ = (0,0).

The outcome of the "stitching" process may be defining a closed volume of facets. To determine the desired island volumetric difference of the closed volume of facets, a sum of signed volumes of tetrahedrons defined by three vertices of each facet may be determined. The origin of the tetrahedrons may be an arbitrary point. The advantage of this method is that the island volumetric difference can be calculated accurately, even if one or more of the island surfaces, on the first or the second 3D dental model, are relatively curved. This may be the case for example, if the island comprises an occlusal edge of an incisor tooth.

To calculate a signed volume of a tetrahedron with vertices $\vec{v_1}, \vec{v_2}, \vec{v_3}$, following formula may be used:

$$\text{Volume} = \frac{1}{6} |\vec{v_1} \cdot (\vec{v_2} \times \vec{v_3})|$$

**In** an embodiment, the island volumetric difference for the island corresponding to the selected list member can be determined in the following manner. For each vertex of the island in the second 3D dental model, a closest distance to the first 3D dental model may be identified, in the direction of the vertex normal. **In** this way back-facing surfaces are excluded. Then, for each facet of the island in the second 3D dental model, an average distance may be calculated based on the identified closest distances of its three vertices and the obtained value may be multiplied by the surface area of that facet. Ultimately, obtained volumes may be summed to determine the island volumetric difference. This approach may be particularly suitable for volume calculation of islands identified on relatively flat teeth surfaces, such as buccal and/or lingual surfaces.

The island volumetric difference may present the user with valuable insight into the overall oral health of the patient because it may precisely quantify change of the dental condition over time. Based on this valuable information, the dental professional may assess the development of the dental condition or its rate of change, and subsequently design an appropriate dental treatment.

**In** one example, the severity factor value determined for the at least one region of the plurality of regions may indicate an absolute change of the at least one dental condition between the first dental 3D scan data of the subject and the second dental 3D scan data of the subject. For example, change in tooth wear for a specific region, expressed in millimeters cubed, can be absolute change. Alternatively or additionally, the severity factor value determined for the at least one region of the plurality of regions may indicate a rate of change of the at least one dental condition between the first dental 3D scan data and the second dental 3D scan data of the subject.

**In** one example the at least first 3D scan data of the subject is obtained by scanning an oral cavity of the subject with an intraoral scanner. This data represents teeth and gingiva of the subject and can be used to generate the 3D dental model.

**In** one embodiment, the method according to disclosure may comprise displaying annotations on the 3D dental model based on user input.

## BRIEF DESCRIPTION OF THE FIGURES

Aspects of the disclosure may be best understood from the following detailed description taken in conjunction with the accompanying figures. The figures are schematic and simplified for clarity, and they just show details to improve the understanding of the claims, while other details are left out. The individual features of each aspect may each be combined with any or all features of the other aspects. These and other aspects, features and/or technical effect will be apparent from and elucidated with a reference to the illustrations described hereinafter in which:

    FIG. 1 illustrates a 3D dental model of lower jaw;
    FIG. 2 illustrates a method according to the disclosure;
    FIG. 3 illustrates a 3D dental model with islands represented by a heat map;
    FIG. 4 illustrates a digital dental chart;
    FIG. 5 illustrates a dental scanning system.

## DETAILED DESCRIPTION

In the following description, reference is made to the accompanying figures, which show by way of illustration how the invention may be practiced.

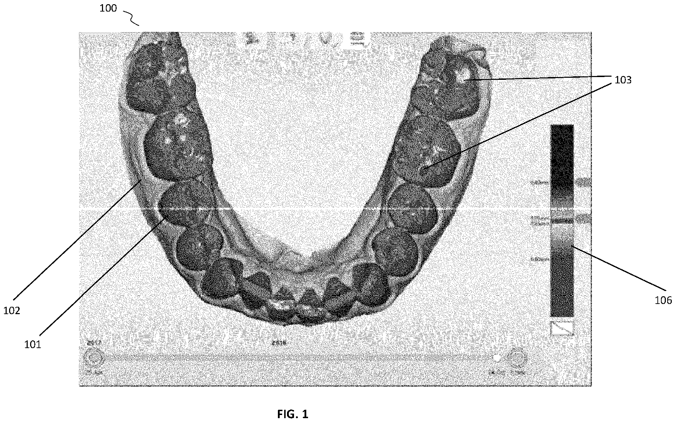

Figure 1 illustrates a 3D dental model 100 of the subject's lower jaw where individual teeth 101 and surrounding gingiva 102 are shown. The term 3D model may refer to a three-dimensional model of teeth and gingiva and may include topological surface information. The 3D dental model may represent lower jaw, upper jaw or both the lower and the upper jaw and can be generated based on received at least a first dental 3D scan data of the subject. The first 3D scan data may be a series of 2D images. The first 3D scan data may comprise texture data such as color data or fluorescence data and may be obtained by scanning oral situation of the subject, for example with an intraoral 3D scanner at a first point in time. This process may also be referred to as obtaining a first scan. In some examples, the series of 2D images may comprise at least one infrared 2D image. A first 3D dental model may then be generated based on the first 3D scan data. In case the first scan is the only obtained scan, the generated 3D dental model is the first 3D dental model. A dental condition, such as caries, may be detected on the 3D dental model 100 generated from a single scan, as will be described later in more detail. The subject, or a patient, may decide to obtain a second scan of the oral situation at a second point in time, later than the first point in time, for example one year later than the first point in time. In that case, a second 3D scan data is obtained, and a second 3D dental model may be generated from the second 3D scan data. The 3D dental model 100, in this case, is generated based on the first and the second 3D scan data. The 3D dental model may be a combination of the first and the second 3D dental model such that the second 3D dental model is superimposed on the first 3D dental model. The first and the second 3D dental models are mutually aligned. The 3D dental model 100 generated based on the first and the second 3D scan data can be used to directly compare the scan data and detect dental conditions such as caries and/or tooth wear. The 3D dental model 100 may also be generated based on more scans than just the first and the second scan.

Using infrared light in teeth scanning process may be advantageous as teeth are highly transparent at infrared wavelengths. Additionally, infrared light can be scattered by caries. This makes infrared light particularly useful for detecting interproximal caries which can otherwise be detected only by X-rays.

The 3D dental model 100 may be represented as a polygonal 3D mesh, most often as a triangular mesh. The triangular mesh is comprised of points, referred to as vertices, and edges connecting the vertices. The edges define faces or facets of the mesh that can be of triangular shape. Alternatively, the 3D dental model may be represented as a point cloud or any other appropriate format for 3D object representation.

Figure 1 also shows islands 103 with a dental condition, in this case tooth wear visible on molar and incisor teeth. Tooth wear may be represented with a visual indicator which may be a heat map as in figure 1. Different colors are used to indicate different severity levels of tooth wear. A vertical bar 106 may serve as a legend to read the heat map and may indicate what color represents what level of tooth wear. Values on the vertical bar 106 can be shown in millimeters and represent the distances between vertices of the aligned first and second 3D dental models.

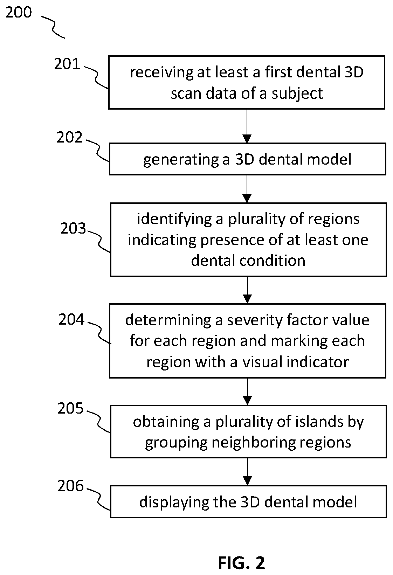

Figure 2 illustrates steps of the computer-implemented method for identifying islands of interest according to the disclosure. The first step 201 of the method comprises receiving at least a first dental 3D scan data of the subject. The dental 3D scan data may be a series of 2D images acquired by an intraoral 3D scanner. The dental 3D scan data may comprise depth map images with information relating to the distance of objects from a viewpoint. The dental 3D scan data may also comprise texture data such as color data and/or fluorescence data.

The at least first 3D dental scan data may then be used to generate the 3D dental model 300 in step 202. The process of generating the 3D dental model 300 which represents subject's teeth and gingiva may comprise reconstructing the series of images into three-dimensional form. In case the second dental 3D scan data has been received in addition to the first dental 3D scan data, then the 3D dental model 300 is generated based on both the first and the second received dental 3D scan data. For example, the first 3D dental model may be generated based on the first dental 3D scan data and the second 3D dental model may be generated based on the second dental 3D scan data. The 3D dental model 300 may then comprise a combination of the first and the second 3D dental model which mutually overlap.

Such overlap of the first and the second 3D dental models may in one example be achieved by applying a commonly used Iterative Closest Point (ICP) algorithm which minimizes the difference between points (vertices) of the first and the second 3D dental model in an iterative manner. This algorithm iteratively revises translation and rotation transformations needed to minimize the distance between the two 3D dental models. The inputs to the algorithm are points' coordinates from the first and the second 3D dental models, i.e., coordinates of vertices, initial estimation of the transformation and the criteria for stopping the iteration. The output of the algorithm is a refined transformation resulting in the 3D dental model 300 comprised of overlapping the first and the second 3D dental model.

Once the 3D dental model 300 is generated, a plurality of regions indicating presence of at least one dental condition is identified in step 203. Dental condition may be, for example, one of caries, tooth wear, dental calculus or gingivitis.

Caries may be detected with the intraoral 3D scanner 525 by scanning the teeth, recording the fluorescence emitted from fluorescent material in cariogenic regions of teeth and processing the obtained scan data. For example, presence of more red light in a section of a tooth, compared to a reference, indicates caries presence. Once generated, the 3D dental 300 model stores, in vertices of the 3D mesh, the texture data, such as fluorescence and/or color data. A region on the 3D dental model 300 with carries may then be identified by comparing fluorescence data in a vertex of the 3D mesh with a reference data representing caries absence. The reference data may be obtained by sampling, for each vertex or facet in the 3D mesh, a value from the texture data and generating a texture value distribution, for example in view of a histogram from the sampled texture values. The texture value distribution may include regions with dental conditions as well as vertices or facets of the 3D mesh that are without dental conditions, i.e. healthy. Filtering may be applied, by defining a minimum threshold for the texture intensity value, for example a scalar value of 15. Such filtering discard regions with dental conditions as they are characterized by low texture values and allows for defining the reference data.

The 3D dental model 300 may be generated based on the first and the second 3D dental models aligned in a same coordinate system. The first and the second 3D dental models may be segmented into different objects such as individual teeth. The alignment may be done on individual corresponding objects of the first and the second 3D dental model by using the mentioned ICP algorithm.

Regions with dental condition such as tooth wear may be identified by calculating distances between corresponding vertices in the first and the second 3D dental models, when the models are aligned. If, for example, the Euclidian distance between the corresponding vertices is above a distance threshold, for example 0,3 mm, then tooth wear is identified for the regions on the 3D dental model 300 comprising the two vertices.

In step 204, the severity factor value for each region of the plurality of regions is determined, wherein the severity factor indicates a level of severity of the at least one dental condition. For example, level of severity of caries or tooth wear may be initial (low), moderate (medium) or severe (high). In case of tooth wear, the distances between corresponding vertices in the first and the second 3D dental models, expressed in millimeters, may be used as severity factor values. In case of caries, output of a scoring function applied to texture data, such as color or fluorescence, may represent the severity factor value. The scoring function may, in one embodiment, be expressed as:

$$f(R,G) = (R-G)/(R+G)$$

where R represents value of Red channel and G represents value of Green channel for a region, obtained from texture data such as fluorescence data of the 3D dental model 300. In the above function, the higher the ratio is, the higher the severity of caries at the corresponding region is. Other scoring functions may be applied, for example comprising only a single texture component of a region, for example Green channel. In another embodiment, the scoring function may comprise texture data of the region with caries and the reference data.

In step 204, each region of the plurality of regions may be marked with a visual indicator, for example a color. A color layer may be overlayed over each region, wherein the color is associated with the severity factor value determined for said region. For example, regions having severity factor values corresponding to the initial (low) severity of a dental condition may be represented in green color. Regions associated with moderate (medium) severity may be represented in orange color and regions associated with high severity may be represented in red color.

In step 205, a plurality of islands 303 is obtained by grouping the plurality of regions on the 3D dental model 300 such that each island 303 comprises neighboring regions indicating presence of at least one dental condition. The term "island" may be understood as a surface area, or an area of interest or a volume on the 3D dental model 300 and can be comprised of neighboring regions. The island 303 on the 3D model 300 may have any shape and be characterized by its surface size and/or its volume. For example, some regions of the island 303 may indicate presence of caries while other regions of the same island may indicate presence of tooth wear. Alternatively, all regions of one island 303 may indicate presence of the same dental condition, such as tooth wear or caries. One common feature all regions of the same island 303 share is that they neighbor at least one other region of that island 303. Therefore, two neighboring regions, which can be two facets of the 3D mesh, share a common edge and/or a common vertex. By grouping the neighboring regions together into islands 303, a significant advantage is achieved because globalized information on subject's dental conditions is created. The algorithm to group the plurality of regions into islands may be following. When a region indicating presence of at least one dental condition is identified then the algorithm may check whether the vertices of the identified region belong to another neighboring region also indicating presence of at least one dental condition. The two regions may then be assigned to a common island.

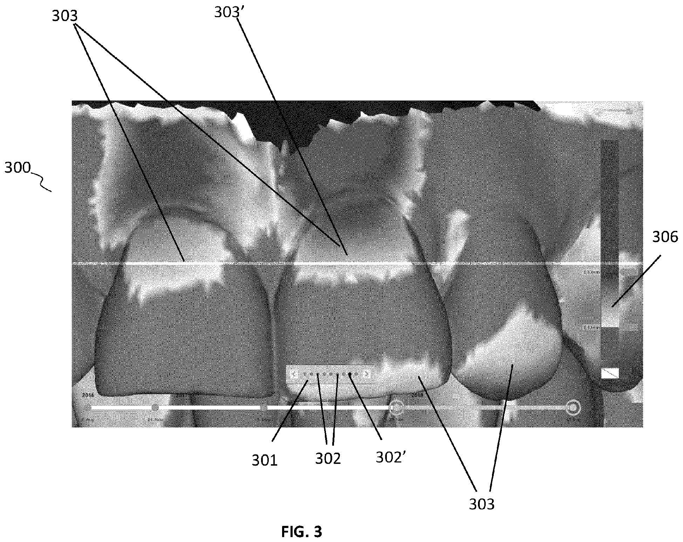

In the step 206 the 3D dental model 300 may be displayed, for example on a display of a laptop or a tablet. In one embodiment the 3D dental model 100 can be displayed, as shown in Fig. 1, such that the complete model fits the display size. In case of Fig. 1, the 3D dental model 100 is a model of the subject's lower jaw, with a view of occlusal plane. In another embodiment, the displaying step may occur such that the 3D dental model 300 may be automatically adjusted for a particular island or group of islands to be shown to the user, as seen in Fig. 3.

The display may be any display suitable for rendering the 3D dental model. Examples include liquid crystal displays (LCDs), light emitting diode (LED) displays and other. The display may include touch screen interface allowing for user interaction with the display.

Figure 3 illustrates a zoomed-in section of a 3D dental model 300, in this case displaying sections of both the upper and lower jaw such that front incisor teeth of the upper jaw are in focus. Figure 3 shows user interface in form of a horizontal bar representing a preview list 301. The preview list 301 may comprise a plurality of list members 302, wherein each list member 302 is associated with an island 303 from the plurality of islands such that the user can navigate between the islands 303 on the 3D dental model 300 by navigating between the list members 302. Fig. 3 illustrates tooth wear in form of a heat map where different levels of severity of tooth wear are indicated with different colors. Vertical bar 306 links the colors used in the heat map to amount of tooth wear expressed in millimeters and may be used to interpret the level of severity of tooth wear.

As shown in Fig. 3, the user can navigate between the different list members 302 by clicking on "Previous" or "Next" button. In this case, the selected list member 302' may correspond to the island 303' on the incisor tooth of the 3D dental model 300. This correspondence between the list members 302 and the islands 303 is advantageous because the user is presented with a finite number of list members associated with each identified dental condition. By navigating through and inspecting all the list members 302, the user can be confident that no identified dental condition is left uninspected. The user may navigate between the different list members 302, alternatively, with a mouse "scroll" button or any other suitable user input method.

If the list member 302' is selected by the user, the 3D dental model 300 may be automatically adjusted such that the island 303', corresponding to the list member 302', is brought into the field of view. This means that the 3D dental model 300 may be rotated, translated, sections of it zoomed in or zoomed out. The island 303' is thus displayed. Additionally, at least a part of the 3D dental model 300 may be made transparent. This may be advantageous if some parts of the 3D dental model 300 block the user's view of the island 303 corresponding to one of the list members 302.

The island 303' associated with the list member 302' may be brought into the field of view as follows. The island 303' can be comprised of the plurality of individual building blocks or regions, each of which indicates presence of at least one dental condition. The region may be a triangle or a facet of a 3D mesh. All vertices of the regions in the island 303' are characterized by their coordinates in the 3D space. It may then be possible to perform a plane fit for all vertices in the regions comprised in the island 303', for example by using least squares method and to identify a normal for the obtained plane. The 3D dental model 300 may then be adjusted so that the normal for the obtained plane coincides with a direction perpendicular to the display surface. In this way the island 303' is brought into the field of view.

At least one infrared (IR) image of a tooth or a plurality of teeth associated with the displayed island 303' may be displayed. This may be advantageous as the user can inspect the at least one infrared image for presence of the dental condition such as caries, in particular interproximal caries formed between the teeth.

The preview list 301 may comprise list members 302 sorted according to a sorting criterion. In one embodiment the sorting criterion may be the island surface area. The island surface area can be calculated as sum of areas of individual regions comprised in the island 303. In case a region is a 3D mesh triangle, then area of the region is calculated according to the formula for calculating triangle area based on known coordinate points. Then, the list member 302 corresponding to the island 303 with the largest surface area may appear as the first list member, a list member 302 corresponding to the island 303 with the second largest surface area may appear as the second list member, and so on. The user would therefore be presented first with the island 303 having the largest island surface area. This means that the island 303 having the largest island surface area would be brought into the field of view. By navigating through the list, for example by clicking "Next" button once, the user would be presented with the island 303 having the second largest island surface area.

The sorting criterion may alternatively be the island significance factor value which can be defined as a function of the island surface area and severity factor values of the regions comprised in the island 303. The island significance factor value may be a scalar value. In one example, the severity factor value of the region in the island 303 may be average distance between vertices of the corresponding facets in the first and the second 3D dental model. This average distance may then be multiplied by the surface area of that region. This process can be repeated for all regions in the island 303. The island significance factor may then be derived as a sum of obtained products of average distances and corresponding region surfaces. In case the average distance is a negative value, it then indicates tooth wear. In some examples it may be beneficial to emphasize tooth wear so that the average distance having a negative value may additionally be multiplied by a weight coefficient, for example a scalar value 1.5. In another example, the island significance factor value may be obtained by multiplying the island surface area with a sum of the severity factor values of the regions comprising the island 303.

A list member 302 corresponding to the island 303 with the highest island significance factor value may appear as the first list member. This list member 302 may be automatically identified and the 3D dental model 300 may be adjusted so that the corresponding island 303 is brought into the field of view. Adjustment of the dental 3D model 300 may be performed automatically. The corresponding island 303 can be brought into the field of view by performing a plane fit for all vertices in the regions comprised in the island 303 and identifying a normal for the obtained plane. The 3D dental model 300 may then be adjusted so that the normal for the obtained plane coincides with a direction perpendicular to the display surface.

In one embodiment it may be possible to generate an optimized travel path when adjusting the 3D dental model 300. In some cases, it may occur that consecutive list members 302 of the sorted preview list 301 correspond to the islands 303 that are relatively distant from each other on the 3D dental model 300. For example, the island 303 corresponding to the first list member 302 and the island 303 corresponding to the second list member 302 may be located on the opposite sides of the 3D dental model 300. Navigation between such list members 302 may be ineffective and confusing to the user as it may be difficult to follow how the 3D dental model 300 is adjusted in the process. Similarly, navigation between islands 303 that are too close to each other on the 3D dental model 300 may also be ineffective as the user may not notice how the 3D dental model 300 is adjusted. In this case, it may be desired that the 3D dental model 300 is first zoomed out, before translations and rotations are performed. Then, the 3D dental model 300 is zoomed in, such that the selected island 303 is brought into the field of view.

By having the optimized travel path, the adjustment of the 3D dental model 300 is improved. When the user navigates between the list members 302 by clicking "Next" button, the 3D dental model 300 can be adjusted such that the island 303, being closest to the previously displayed island, is brought into the field of view. This means that one or more of the list members 302 may be rearranged in order to avoid large adjustment of the 3D dental model, i.e. significant rotations and translations. The optimized travel path may be created by rearranging the list members 302 in the preview list 301. For example, the preview list 301 may be rearranged such that second list member 302 and a third list member 302 change places in the preview list 301 if the island 303, associated to the third list member 302, is the closest to the island 302 corresponding to the first list member 302 on the 3D dental model 300. Advantage of this approach is that minimum amount of adjustment of the 3D dental model 300 is performed, i.e. minimum amount of rotation and translation between individual navigation steps. The rearrangement of the list members 302 may be performed automatically, based on a rearrangement criterion. In the abovementioned case, the rearrangement criterion is the distance between the islands 303 on the 3D dental model 300.

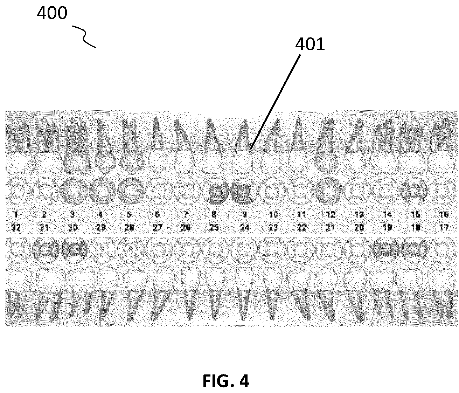

Figure 4 is illustrative of a digital dental chart 400. Parameters such as the island significance factor value or the island surface area may be copied into the digital dental chart 400. The digital dental chart 400 may comprise information on identified dental conditions associated with tooth representations 401. For example, the island surface area may be displayed indicating the surface affected by caries. Different tooth representations 401 in the digital dental chart 400 may be colored according to colors used in marking the 3D dental model 300. The digital dental chart 400 is represented in format of Universal Numbering System but other formats can be applied as well, for example FDI World Dental Federation (ISO) notation or Palmer notation.

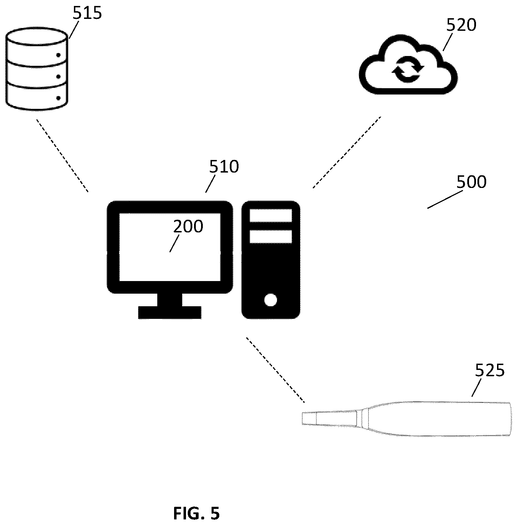

FIG. 5 illustrates a dental scanning system 500 which comprises means for carrying out the method 200. The dental scanning system 500 may comprise a computer 510 which may have a wired or wireless interface to a server 515, a cloud server 520 and an intraoral scanner 525.

The intraoral scanner 525 may be capable of recording the at least first and second 3D dental scan data of the subject. The recorded at least first and second 3D dental scan data may comprise geometrical and texture data for the teeth. The texture data may include color and/or fluorescence data.

Additionally, the intraoral scanner 525 may be equipped with an infrared module and capable of recording infrared data. The infrared module may comprise an infrared light source. The infrared data can be recorded at infrared wavelengths, for example at near infrared wavelengths of 800-2500 nm. The infrared data may be in form of infrared 2D images.

Infrared 2D images may be correlated with the 3D dental model 100, 300 and may be displayed together with displaying the 3D dental model 100, 300.

The dental scanning system 500 may comprise a data processing device configured to carry out the method according to one or more embodiments of the disclosure. The data processing device may be a part of the computer 510.

A non-transitory computer-readable storage medium may comprise instructions which, when executed by a computer, cause the computer to carry out the method according to one or more embodiments of the disclosure.

Additionally or alternatively, the non-transitory computer-readable storage medium may comprise instructions which, when executed by a computer, cause the computer to render a graphical user interface that is configured to load and visualize the 3D dental model 100, 300 with the plurality of islands 303. The computer may additionally render the preview list 301. The preview list 301 may comprise a plurality of list members 302, wherein each list member 302 is associated with an island 303 from the plurality of islands such that the user can navigate between the islands 303 on the 3D dental model 300 by navigating between the list members 302.

A computer program product may be embodied in the non-transitory computer readable medium. The computer program product may comprise instructions which, when executed by a computer, cause the computer to perform the method according to any of the embodiments presented herein.

Although some embodiments have been described and shown in detail, the disclosure is not restricted to such details, but may also be embodied in other ways within the scope of the subject matter defined in the following claims.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

**Claims**

1. Computer-implemented method for identifying islands (303) of interest on a 3D dental model (100, 300), the method comprising the steps:

    - receiving at least a first and a second dental 3D scan data of a subject, the second dental 3D scan data being obtained later in time compared to the first dental 3D scan data;
    - generating the 3D dental model (100, 300) based on the received at least first and second dental 3D data of the subject;
    - identifying a plurality of regions on the 3D dental model (100, 300), each region of the plurality of regions indicating presence of at least one dental condition;
    - determining a severity factor value for each region of the plurality of regions, the severity factor value indicating a level of severity of the at least one dental condition;
    - marking, on the 3D dental model (100, 300), each region of the plurality of regions with a visual indicator, wherein the visual indicator is selected based on the determined severity factor value for said region;
    - obtaining a plurality of islands (303) by grouping the plurality of regions on the 3D dental model (100, 300) such that each island comprises neighboring regions indicating presence of the at least one dental condition;

- displaying the 3D dental model (100, 300) with the plurality of islands (303), **characterised in that** the method further comprises:
- displaying a user interface in form of a preview list (301), the preview list (301) comprising a plurality of list members (302), wherein each list member (302) is associated with an island (303) from the plurality of islands (303) such that a user can navigate between the islands (303) on the 3D dental model (100, 300) by navigating between the list members (302), further comprising when a list member (302') from the preview list (301) is selected by the user:
- automatically adjusting the 3D dental model (100, 300) so that the island (303') corresponding to the selected list member (302') is brought into the field of view;
- calculating an island volumetric difference value for the island (303') corresponding to the selected list member (302') and
- displaying the island volumetric difference value for the island (303') corresponding to the selected list member (302').

2. The method according to claim 1, wherein the at least one dental condition is caries and wherein identifying the plurality of regions on the 3D dental model (100, 300), each region of the plurality of regions indicating presence of caries, comprises:

   - comparing fluorescence data in a vertex of a 3D mesh of the 3D dental model (100, 300) with a reference data representing caries absence.

3. The method according to the previous claim 2, wherein the reference data representing caries absence is obtained by:

   - for each vertex or facet in the 3D mesh sampling a value from texture data;
   - generating a texture value distribution including regions with caries and healthy regions;
   - defining a minimum threshold for the texture value distribution to separate regions with caries and healthy regions.

4. The method according to any previous claim, wherein the at least one dental condition is tooth wear and wherein identifying the plurality of regions on the 3D dental model (100, 300), each region of the plurality of regions indicating presence of tooth wear, comprises:

   - calculating distances between corresponding vertices in the first and the second 3D dental models when the models are aligned;
   - if an Euclidian distance between the corresponding vertices is above a distance threshold, identifying tooth wear for the regions on the 3D dental model (100, 300) comprising the corresponding vertices.

5. The method according to any previous claim, wherein the regions of the island (303') indicate presence of the same dental condition.

6. The method according to any previous claim 1 to 5, wherein the regions of the island (303') indicate presence of different dental conditions.

7. The method according to any previous claim, wherein grouping the plurality of regions on the 3D dental model (100, 300) such that each island (303') comprises neighboring regions indicating presence of the at least one dental condition comprises:

   - identifying a region indicating presence of the at least one dental condition;
   - assessing whether vertices of the identified region belong to another neighboring region also indicating presence of the at least one dental condition;
   - assigning the region and the neighboring region to the island (303') based on the assessment.

8. The method according to any previous claim, wherein automatically adjusting the 3D dental model (100, 300) comprises fading out the part of the 3D dental model (100, 300) other than the island (303') brought into the field of view.

9. The method according to any previous claim, wherein each region is a facet and wherein the island volumetric difference for the island (303') is obtained by:

- calculating signed volumes of tetrahedrons which are formed by joining the vertices of facets in the island (303') with an arbitrary point and summing up the calculated volumes.

10. The method according to any previous claim, wherein generating the 3D dental model (100, 300) based on the received at least first and second dental 3D data of the subject comprises:

- generating a first 3D dental model based on the first dental 3D scan data;
- generating a second 3D dental model based on the second dental 3D scan data;
- overlapping the first and the second 3D dental model.

11. The method according to any previous claim, further comprising automatically sorting the list members (302) in the preview list (301) based on a sorting criterion, wherein the sorting criterion is an island surface area and/or an island significance factor value.

12. The method according to any previous claim further comprising generating an optimized travel path when adjusting the 3D dental model (100, 300).

13. A computer program product embodied in a non-transitory computer readable medium, the computer program product comprising instructions which, when executed by a computer, cause the computer to carry out the method according to any of the claims 1 to 12.

14. A non-transitory computer-readable medium comprising instructions which, when executed by a computer, cause the computer to carry out the method according to any of the claims 1 to 12.

15. A dental scanning system (500) comprising a data processing device configured to carry out the method according to any of the claims 1 to 12.

**Patentansprüche**

1. Computerimplementiertes Verfahren zum Identifizieren von Inseln (303) von Interesse auf einem 3D-Zahnmodell (100, 300), wobei das Verfahren die folgenden Schritte umfasst:

- Empfangen mindestens erster und zweiter dentaler 3D-Scandaten eines Subjekts, wobei die zweiten dentalen 3D-Scandaten zeitlich später im Vergleich mit den ersten dentalen 3D-Scandaten erhalten werden;
- Erzeugen des 3D-Zahnmodells (100, 300) auf Grundlage der empfangenen mindestens ersten und zweiten 3D-Zahndaten des Subjekts;
- Identifizieren einer Vielzahl von Bereichen auf dem 3D-Zahnmodell (100, 300), wobei jeder Bereich der Vielzahl von Bereichen das Vorhandensein mindestens einer Zahnerkrankung anzeigt;
- Ermitteln eines Schwerefaktorwertes für jeden Bereich der Vielzahl von Bereichen, wobei der Schwerefaktorwert einen Schweregrad der mindestens einen Zahnerkrankung angibt;
- Markieren, auf dem 3D-Zahnmodell (100, 300), jedes Bereichs der Vielzahl von Bereichen mit einem visuellen Indikator, wobei der visuelle Indikator auf Grundlage des für den Bereich ermittelten Schwerefaktorwertes ausgewählt wird;
- Erhalten einer Vielzahl von Inseln (303) durch Gruppieren der Vielzahl von Bereichen auf dem 3D-Zahnmodell (100, 300) so, dass jede Insel benachbarte Bereiche umfasst, die das Vorhandensein mindestens einer Zahnerkrankung anzeigen;
- Anzeigen des 3D-Zahnmodells (100, 3 00) mit der Vielzahl von Inseln (3 03), **dadurch gekennzeichnet, dass** das Verfahren weiter Folgendes umfasst:

- Anzeigen einer Benutzeroberfläche in Form einer Vorschauliste (301), wobei die Vorschauliste (301) eine Vielzahl von Listenelementen (302) umfasst, wobei jedes Listenelement (302) einer Insel (303) von der Vielzahl von Inseln (303) zugeordnet ist, so dass ein Benutzer zwischen den Inseln (303) auf dem 3D-Zahnmodell (100, 300) navigieren kann, indem er zwischen den Listenelementen (302) navigiert, wobei es weiter umfasst, wenn ein Listenelement (302') aus der Vorschauliste (301) vom Benutzer ausgewählt wird:

- automatisches Anpassen des 3D-Zahnmodells (100, 300) so, dass die Insel (303'), die dem ausgewählten Listenelement (302') entspricht, ins Sichtfeld gebracht wird;

- Berechnen eines Inselvolumen-Differenzwertes für die Insel (303'), die dem ausgewählten Listenelement (302') entspricht, und
- Anzeigen des Inselvolumen-Differenzwertes für die Insel (303'), die dem ausgewählten Listenelement (302') entspricht.

**2.** Verfahren nach Anspruch 1, wobei die mindestens eine Zahnerkrankung Karies ist und wobei das Identifizieren der Vielzahl von Bereichen auf dem 3D-Zahnmodell (100, 300), wobei jeder Bereich von der Vielzahl von Bereichen das Vorhandensein von Karies anzeigt, Folgendes umfasst:

- Vergleichen von Fluoreszenzdaten in einem Eckpunkt eines 3D-Netzes des 3D-Zahnmodells (100, 300) mit Referenzdaten, die Kariesfreiheit darstellen.

**3.** Verfahren nach dem vorstehenden Anspruch 2, wobei die Referenzdaten, welche die Kariesfreiheit darstellen, durch Folgendes erhalten werden:

- für jeden Eckpunkt oder jede Facette im 3D-Netz Abtasten eines Wertes aus Texturdaten;
- Erzeugen einer Texturwertverteilung einschließlich von Bereichen mit Karies und gesunden Bereichen;
- Definieren eines Mindestschwellenwertes für die Texturwertverteilung, um Bereiche mit Karies und gesunde Bereiche zu trennen.

**4.** Verfahren nach einem vorstehenden Anspruch, wobei die mindestens eine Zahnerkrankung Zahnabrieb ist und wobei Identifizieren der Vielzahl von Bereichen auf dem 3D-Zahnmodell (100, 300), wobei jeder Bereich von der Vielzahl von Bereichen das Vorhandensein von Zahnabrieb anzeigt, Folgendes umfasst:

- Berechnen von Abständen zwischen entsprechenden Eckpunkten in dem ersten und dem zweiten 3D-Zahnmodell, wenn die Modelle ausgerichtet sind;
- falls ein euklidischer Abstand zwischen den entsprechenden Eckpunkten über einem Abstandsschwellenwert liegt, Identifizieren von Zahnabrieb für die Bereiche auf dem 3D-Zahnmodell (100, 300), welche die entsprechenden Eckpunkte umfassen.

**5.** Verfahren nach einem vorstehenden Anspruch, wobei die Bereiche der Insel (303') das Vorhandensein derselben Zahnerkrankung anzeigen.

**6.** Verfahren nach einem der vorstehenden Ansprüche 1 bis 5, wobei die Bereiche der Insel (303') das Vorhandensein unterschiedlicher Zahnerkrankungen anzeigen.

**7.** Verfahren nach einem der vorstehenden Ansprüche, wobei Gruppieren der Vielzahl von Bereichen auf dem 3D-Zahnmodell (100, 300) so, dass jede Insel (303') benachbarte Bereiche umfasst, die das Vorhandensein mindestens einer Zahnerkrankung anzeigen, Folgendes umfasst:

- Identifizieren eines Bereichs, der das Vorhandensein mindestens einer Zahnerkrankung anzeigt;
- Beurteilen, ob Eckpunkte des identifizierten Bereichs zu einem anderen benachbarten Bereich gehören, der ebenfalls das Vorhandensein mindestens einer Zahnerkrankung anzeigt;
- Zuordnen des Bereichs und des benachbarten Bereichs zur Insel (303') auf Grundlage der Beurteilung.

**8.** Verfahren nach einem vorstehenden Anspruch, wobei automatisches Anpassen des 3D-Zahnmodells (100, 3 00) Ausblenden des Teils des 3D-Zahnmodells (100, 300) umfasst, der nicht die Insel (303') ist, die in das Sichtfeld gebracht wurde.

**9.** Verfahren nach einem vorstehenden Anspruch, wobei jeder Bereich eine Facette ist und wobei die Inselvolumen-Differenz für die Insel (303') durch Folgendes erhalten wird:

- Berechnen signierter Volumina von Tetraedern, die durch Verbinden der Eckpunkte von Facetten in der Insel (303') mit einem beliebigen Punkt gebildet werden, und Aufsummieren der berechneten Volumina.

**10.** Verfahren nach einem vorstehenden Anspruch, wobei Erzeugen des 3D-Zahnmodells (100, 300) auf Grundlage der erhaltenen mindestens ersten und zweiten 3D-Zahndaten des Subjekts Folgendes umfasst:

- Erstellen eines ersten 3D-Zahnmodells auf Grundlage der ersten dentalen 3D-Scandaten;
- Erstellen eines zweiten 3D-Zahnmodells auf Grundlage der zweiten dentalen 3D-Scandaten;
- Überlappen des ersten und des zweiten 3D-Zahnmodells.

11. Verfahren nach einem vorstehenden Anspruch, weiter umfassend automatisches Sortieren der Listenelemente (302) in der Vorschauliste (301) auf Grundlage eines Sortierkriteriums, wobei das Sortierkriterium eine Inseloberfläche und/oder ein Inselsignifikanzfaktorwert ist.

12. Verfahren nach einem vorstehenden Anspruch, weiter umfassend Erzeugen eines optimierten Verfahrweges, wenn das 3D-Zahnmodell (100, 300) angepasst wird.

13. Computerprogrammprodukt, das in einem nichtflüchtigen computerlesbaren Medium verkörpert ist, wobei das Computerprogrammprodukt Anweisungen umfasst, die, wenn sie von einem Computer ausgeführt werden, den Computer veranlassen, das Verfahren nach einem der Ansprüche 1 bis 12 durchzuführen.

14. Nichtflüchtiges, computerlesbares Medium, das Anweisungen umfasst, welche, wenn sie von einem Computer ausgeführt werden, den Computer veranlassen, das Verfahren gemäß einem der Ansprüche 1 bis 12 durchzuführen.

15. Dentales Scansystem (500), das eine Datenverarbeitungsvorrichtung umfasst, die so konfiguriert ist, dass sie das Verfahren nach einem der Ansprüche 1 bis 12 durchführt.

**Revendications**

1. Procédé mis en œuvre par ordinateur pour identifier des îlots (303) d'intérêt sur un modèle dentaire 3D (100, 300), le procédé comprenant les étapes consistant à :

- recevoir au moins des premières et des secondes données de balayage dentaire 3D d'un sujet, les secondes données de balayage dentaire 3D étant obtenues plus tard dans le temps par comparaison aux premières données de balayage dentaire 3D;
- générer le modèle dentaire 3D (100, 300) sur la base des au moins premières et secondes données dentaires 3D reçues du sujet ;
- identifier une pluralité de régions sur le modèle dentaire 3D (100, 300), chaque région de la pluralité de régions indiquant la présence d'au moins une condition dentaire ;
- déterminer une valeur de facteur de gravité pour chaque région de la pluralité de régions, la valeur du facteur de gravité indiquant un niveau de gravité de la au moins une condition dentaire ;
- marquer, sur le modèle dentaire 3D (100, 300), chaque région de la pluralité de régions avec un indicateur visuel, dans lequel l'indicateur visuel est sélectionné sur la base de la valeur de facteur de gravité déterminée pour ladite région ;
- obtenir une pluralité d'îlots (303) en regroupant la pluralité de régions sur le modèle dentaire 3D (100, 300) de telle sorte que chaque îlot comprenne des régions voisines indiquant la présence de la au moins une condition dentaire ;
- afficher le modèle dentaire 3D (100, 300) avec la pluralité d'îlots (303), **caractérisé en ce que** le procédé comprend en outre les étapes consistant à :

- afficher une interface utilisateur sous forme de liste de prévisualisation (301), la liste de prévisualisation (301) comprenant une pluralité d'éléments de liste (302), dans lequel chaque élément de liste (302) est associé à un îlot (303) parmi la pluralité d'îlots (303) de sorte qu'un utilisateur puisse naviguer entre les îlots (303) sur le modèle dentaire 3D (100, 300) en naviguant entre les éléments de liste (302), comprenant en outre, lorsqu'un élément de liste (302') de la liste de prévisualisation (301) est sélectionné par l'utilisateur, les étapes consistant à :

- ajuster automatiquement le modèle dentaire 3D (100, 300) afin que l'îlot (303') correspondant à l'élément de liste sélectionné (302') soit amené dans le champ de vision ;
- calculer une valeur de différence volumétrique d'îlot pour l'îlot (303') correspondant à l'élément de liste sélectionné (302') et
- afficher la valeur de différence volumétrique d'îlot pour l'îlot (303') correspondant à l'élément de liste sélectionné (302').

**2.** Procédé selon la revendication 1, dans lequel la au moins une condition dentaire est une carie et dans lequel l'identification de la pluralité de régions sur le modèle dentaire 3D (100, 300), chaque région de la pluralité de régions indiquant la présence d'une carie, comprend l'étape consistant à :

- comparer des données de fluorescence dans un sommet d'un maillage 3D du modèle dentaire 3D (100, 300) avec des données de référence représentant l'absence de carie.

**3.** Procédé selon la revendication 2 précédente, dans lequel les données de référence représentant l'absence de carie sont obtenues par :

- pour chaque sommet ou facette du maillage 3D, l'échantillonnage d'une valeur à partir des données de texture ;
- la génération d'une distribution de valeur de texture incluant des régions cariées et des régions saines ;
- la définition d'un seuil minimal pour la distribution de valeur de texture afin de séparer les régions cariées et les régions saines.

**4.** Procédé selon une quelconque revendication précédente, dans lequel la au moins une condition dentaire est l'usure dentaire et dans lequel l'identification de la pluralité de régions sur le modèle dentaire 3D (100, 300), chaque région de la pluralité de régions indiquant la présence d'usure dentaire, comprend les étapes consistant à :

- calculer des distances entre des sommets correspondants dans les premier et second modèles dentaires 3D lorsque les modèles sont alignés ;
- si une distance euclidienne entre les sommets correspondants est supérieure à un seuil de distance, identifier l'usure dentaire pour les régions du modèle dentaire 3D (100, 300) comprenant les sommets correspondants.

**5.** Procédé selon une quelconque revendication précédente, dans lequel les régions de l'îlot (303') indiquent la présence de la même condition dentaire.

**6.** Procédé selon une quelconque revendication 1 à 5 précédente, dans lequel les régions de l'îlot (303') indiquent la présence de différentes conditions dentaires.

**7.** Procédé selon une quelconque revendication précédente, dans lequel le regroupement de la pluralité de régions sur le modèle dentaire 3D (100, 300) de sorte que chaque îlot (303') comprenne des régions voisines indiquant la présence de la au moins une condition dentaire comprend les étapes consistant à :

- identifier une région indiquant la présence de la au moins une condition dentaire ;
- évaluer si les sommets de la région identifiée appartiennent à une autre région voisine indiquant également la présence de la au moins une condition dentaire ;
- attribuer la région et la région voisine à l'îlot (303') sur la base de l'évaluation.

**8.** Procédé selon une quelconque revendication précédente, dans lequel l'ajustement automatique du modèle dentaire 3D (100, 300) comprend l'estompage de la partie du modèle dentaire 3D (100, 300) autre que l'îlot (303') amenée dans le champ de vision.

**9.** Procédé selon une quelconque revendication précédente, dans lequel chaque région est une facette et dans lequel la différence volumétrique d'îlot pour l'îlot (303') est obtenue par :

- le calcul de volumes signés des tétraèdres formés en reliant les sommets des facettes de l'îlot (303') avec un point arbitraire et en additionnant les volumes calculés.

**10.** Procédé selon une quelconque revendication précédente, dans lequel la génération du modèle dentaire 3D (100, 300) sur la base des au moins premières et secondes données dentaires 3D reçues du sujet comprend les étapes consistant à :

- générer un premier modèle dentaire 3D sur la base des premières données de balayage dentaire 3D ;
- générer un second modèle dentaire 3D basé sur les secondes données de balayage dentaire 3D ;
- superposer les premier et second modèles dentaires 3D.

**11.** Procédé selon une quelconque revendication précédente, comprenant en outre le tri automatique des éléments de liste (302) dans la liste de prévisualisation (301) sur la base d'un critère de tri, dans lequel le critère de tri est la surface d'îlot et/ou une valeur de facteur de signification d'îlot.

12. Procédé selon une quelconque revendication précédente, comprenant en outre la génération d'un trajet de déplacement optimisé lors de l'ajustement du modèle dentaire 3D (100, 300).

13. Produit de programme informatique incorporé dans un support non transitoire lisible par ordinateur, le produit de programme informatique comprenant des instructions qui, lorsqu'elles sont exécutées par un ordinateur, amènent l'ordinateur à exécuter le procédé selon l'une quelconque des revendications 1 à 12.

14. Support non transitoire lisible par ordinateur comprenant des instructions qui, lorsqu'elles sont exécutées par un ordinateur, amènent l'ordinateur à exécuter le procédé selon l'une quelconque des revendications 1 à 12.

15. Système de balayage dentaire (500) comprenant un dispositif de traitement de données configuré pour exécuter le procédé selon l'une quelconque des revendications 1 à 12.

**FIG. 1**

200

201 — receiving at least a first dental 3D scan data of a subject

202 — generating a 3D dental model

203 — identifying a plurality of regions indicating presence of at least one dental condition

204 — determining a severity factor value for each region and marking each region with a visual indicator

205 — obtaining a plurality of islands by grouping neighboring regions

206 — displaying the 3D dental model

**FIG. 2**

**FIG. 3**

FIG. 4

515

520

510

200

500

525

**FIG. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020182880 A1
- US 2012268463 A1
- US 2017308626 A1
- US 2013271462 A1
- US 2008118131 A1
- WO 2021113501 A
- US 2021128282 A1

**Non-patent literature cited in the description**

- **THOMAS FORGIONE et al.** Impact of 3D bookmarks on navigation and streaming in a networked virtual environment. *PROCEEDINGS OF THE 7TH INTER-NATIONAL CONFERENCE ON MULTIMEDIA SYS-TEMS, MMSYS '16*, 10 May 2016